# EUROPEAN PATENT APPLICATION

(11) **EP 1 005 835 A1**
(43) Date of publication of application: **07.06.2000**
(21) Application number: 99119034.9
(22) Date of filing: 29.09.1999
(51) Int. Cl.: A61B 8/12, A61B 8/08

(54) **System for three-dimensional imaging of an internal organ or body structure**

(30) Priority: 01.12.1998 SE 9804147
(71) Applicant: Siemens-Elema AB, 171 95 Solna 1 (SE)
(72) Inventor: Ubby, Johan, West Port, CT 06880 (US)

(57) **Abstract**

A system for 3-dimensional imaging of an internal organ (12) or body structure of a patient (6), comprising an ultrasound probe (10), equipped with localization sensors (14) intended for application to the exterior of the patient in order to generate an ultrasonograph of the organ or body structure. A localization unit (16) is arranged to determine the position of the probe in relation to a reference (2, 4), and an image generation unit (18) is arranged to generate a three-dimensional image of the organ from a plurality of ultrasonographs taken with the probe in different positions and from associated information from the localization unit on the probe's position. At least one moveable reference sensor (4) is devised to be placed in a fixed position in relation to the said organ or body structure to be visualized in order to form the said reference.

## Description

The present invention relates to a system for three-dimensional imaging of an internal organ or body structure of a patient, comprising an external ultrasound probe equipped with localization sensors intended for application to the exterior of the patient and generating an ultrasonograph of the organ or body structure, a localization unit arranged to determine the position of the probe in relation to a reference and an image-generating unit arranged to generate a three-dimensional image of the organ from a plurality of ultrasonographs taken with the probe in different positions and from associated information from the localization unit on the probe's position.

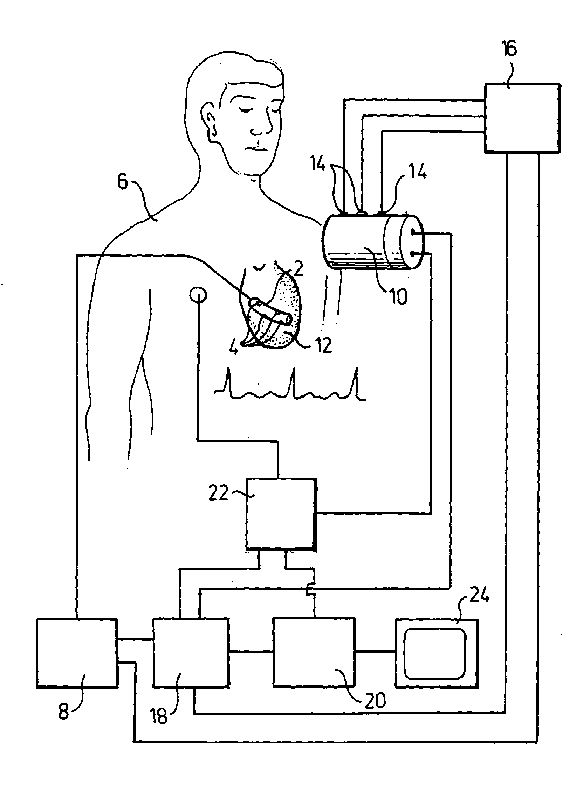

Generation of a three-dimensional image of an internal organ or body structure from a plurality of 2-dimensional images captured with an external ultrasound probe is previously known from e.g. US-A-5,078,145 and 5,353,354. In this technology, the position of the ultrasound probe in relation to the organ or body structure to be visualized must be determined for each image recorded. Thus, US-A-5,078,145 describes a device in which the ultrasound probe is attached to one end of a robot arm mounted on the examination table on which the patient is to lie during the examination (See FIG. 4 and related text in the description thereof). With this mechanical solution, the position of the ultrasound probe can therefore be determined relative to the examination table and, accordingly, to a patient lying on that examination table. US-A-5,353,354 describes one embodiment in which accelerometers, arranged in the ultrasound probe, are employed for determining the position of the ultrasound head or the probe in relation to a specific starting position, and one embodiment with a probe localization system based on magnetic field detection. This latter localization system comprises a magnetic field sensor, attached to the bed on which the patient is to lie during the ultrasound examination, and the ultrasound probe is equipped with a receiver. The position of the currently recorded two-dimensional image relative to a preselected reference plane is determined from the signals from the magnetic field detector and the transmitter. Thus, the imaging systems according to these two American patent documents comprise localization equipment installed in the examination room or mounted on the examination bed (table).

The disadvantage of these prior art imaging systems with a fixed reference in the room or relative to the examination bed is that detailed three-dimensional studies of a moving organ or structure, such as the heart of a patient, are not possible. Since the heart is in continuous motion inside the body because of its intrinsic activity and even because of the patient's breathing and possible body movements, an accurate determination of the heart's structure cannot be made using this prior art technique.

Another kind of system for identifying the position of a measurement and/or treatment catheter in a vessel or organ of a patient is described in WO 98/00060, whereby a reference catheter is devised for introduction into e.g. the heart. This reference catheter serves as a reference for a measurement or treatment catheter, also inserted into the heart, normally intended for introduction into a chamber of the heart. The reference catheter does not therefore serve as a reference for any external instrument. The publication specifically states that the reference catheter and measurement and/or treatment catheter should be arranged close to each other, so relative movement between them is minimized when the heart moves.

The objective of the present invention is to achieve a system of the aforesaid kind for three-dimensional imaging of an internal organ or body structure of a patient with the aid of an external ultrasound probe, said system being highly suitable even for obtaining images of the detailed structure of organs in motion.

This objective is achieved with a system of the aforesaid kind with the features set forth in claim 1.

When at least one moveable reference sensor is devised to be in a fixed position in relation to the said organ or body structure, thereby forming the said reference, interference caused by movement of the organ or body structure is eliminated, since the reference sensor in the system according to the invention is compliant with the movements of the organ or body structure.

In principle, the system according to the invention can be used for visualizing any organ or any body structure, and the system according to the invention offers a simple and fast procedure for, for example, displaying a three-dimensional image of the heart and its chambers on a computer screen, a capability long sought. In this manner, one of the most cost-effective tools for the diagnosis and treatment of heart patients is achieved. Thus, congenital anomalies of the heart, for example, can be easily diagnosed. Shunt and valve problems can also be diagnosed more accurately than has hitherto been possible, and the time needed for ablation procedures and procedures for determining arrhythmia in electrophysiological studies can be drastically reduced.

According to one advantageous embodiment of the system according to the invention, the reference sensor is mounted on a reference catheter, designed for insertion to a fixed position in the organ or body structure to be visualized. In visualization of the heart, for example, the reference catheter is suitably placed in the coronary sinus, since this is generally compliant with the beats of the heart and heart movements such as are caused by the patient's breathing. The reference sensor is accordingly compliant with the heart's movements.

According to another advantageous embodiment of the system according to the invention, the probe is equipped with at least three separate localization sensors, thereby making possible three-dimensional localization of the probe's position.

According to another advantageous embodiment of the system according to the invention, the localization sensors are arranged on the probe in a single, essentially straight line which, when the probe is applied in the measurement position on the patient, is largely perpendicular to the probe's contact surface with the patient. This design makes possible particularly accurate determination of the probe's position.

According to yet another advantageous embodiment of the system according to the invention, four separate reference sensors are arranged on the reference catheter. Three of the sensors are on one plane, e.g. an xy plane, and the fourth reference sensor is used for determining the z axis. In this way, a complete three-dimensional coordinate system is defined which is compliant with the movements of the organ or body structure and in which the position of the probe's localization sensors and, accordingly, the position of the probe are identified.

According to another advantageous embodiment of the system according to the invention, each reference and localization sensor holds an ultrasound transceiver and localization unit that are devised to determine the position of the probe in relation to the reference sensor(s) from the signal from the reference and localization sensors. Thus, the same kind of sensors can be used advantageously as reference or localization sensors respectively, and the sensors can suitably comprise ultrasound crystals for measuring the distance between the reference and localization sensors, based on measurement of the transit time for ultrasound pulses. This has proved to be a simple and reliable method.

According to additional advantageous embodiments of the system according to the invention, image storage means are arranged for storing the three dimensional image of the organ, and an ECG device is arranged to trigger the image generating unit and the said image storage means in order to store the images of ultrasonographs taken at a particular point in the cardiac cycle. When characteristic parts of the ECG are employed for triggering purposes, images of the heart can be generated at different phases of the cardiac cycle. This feature is often useful.

In order to explain the invention in greater detail, an exemplifying embodiment of the system according to the invention will be described below in greater detail, referring to the attached drawing which is a block diagram of the embodiment as applied to a patient.

A reference catheter 2 with a reference sensor 4 have accordingly been introduced into the coronary sinus of a patient 6. The reference catheter's 2 sensor 4 is connected to a reference measurement unit 8 which is arranged to controllably feed the reference sensors 4.

An ultrasound probe 10 of the known kind is devised for application to the exterior of the patient 6 to generate an echocardiographic, two-dimensional ultrasonographic image of the organ or body structure being studied, the heart 12 in this instance. A number of localization sensors 14 are arranged on the ultrasound probe 10. They are connected to a localization unit 16. When the localization sensors 14 are suitably positioned, the probe's 10 distance, aiming direction and rotational position can be determined in relation to the reference. The unit 16 feeds the localization sensors 14 in the required fashion and receives signals from them for thereby determining the position of the probe 10 in relation to the reference 2, 4.

The reference and localization sensors 2, 14 are suitably of the same kind and contain crystal-based ultrasound transceivers, and the position of the probe 10 in relation to the reference 2, 4 is determined by measuring the distances between the reference and localization sensors 2 and 14 respectively by measuring the transit time for emitted ultrasonic pulses according to a technique sold by the Sonometrics Corporation.

Image information from a plurality of two-dimensional images recorded with the ultrasound probe 10 are sent to an image generating unit 18 with information from the localization unit 16 on the positions of the probe 10 at the time each image was recorded. The image generating unit 18 is arranged to use this information for generating a three-dimensional image of the heart 12 in the known manner.

Image storage means 20 are connected to the image generating unit 18 for storing the generated three-dimensional images.

An ECG device 22 is appropriately arranged to trigger the probe 10, the image generating unit 18 and the image storage means 20, causing them to generate and store three-dimensional images of the heart at optional points in the cardiac cycle. Here, various characteristic parts of the ECG can be used for triggering, e.g. the beginning of the QRS complex, the R spike etc.

The image recording procedure in the system according to the invention is therefore as follows:

The ultrasound probe 10 is placed against the patient's 6 skin in a position at which the heart 12 is visualized. The probe 10 is appropriately applied essentially perpendicular to the thorax. The probe 10 is moved slightly at every heart beat in order to capture a new two-dimensional image for transmission to the image generating unit 18. In this unit 18 an iterative technique is employed for calculating a three-dimensional image of the heart for storage in the image storage unit 20. The image could also be allowed to gradually appear on a computer monitor 24 during calculations. The greater the number of two-dimensional images captured by the probe 10 for generating the three-dimensional image, the greater the clarity and detail of the three-dimensional image obtained.

Since a detailed three-dimensional image of the heart can be obtained quickly and simply in this way, direct study of e.g. the movements of heart valves, and simple introduction of e.g. catheters for imaging, ablation, pressure measurement etc. become possible, and the catheters can be advanced to the exact position desired with no need to move the reference catheter and no need for fluoroscopic imaging.

The invention has been described above as primarily applied to the heart, but the system according to the invention can obviously be used for obtaining images of any internal organ or body structure, including e.g. blood vessels, various abdominal organs etc.

## Claims

1. A system for three-dimensional imaging of an internal organ (12) or body structure of patient (6), comprising an ultrasound probe (10) equipped with localization sensors (14), intended for application to the exterior of the patient in order to generate an ultrasonograph of the organ (12) or body structure, a localization unit (16) arranged to determine the position of the probe relative to a reference (2, 4) and an image generating unit (18) arranged to generate a three-dimensional image of the organ from a plurality of ultrasonographs captured with the probe in different positions and from associated information from the localization unit on the position of the probe, **characterized in** that at least one moveable reference sensor (4) is devised to be placed in a fixed position in relation to the said organ (12) or body structure to be visualized so as to form the said reference.

2. The system according to claim 1, **characterized in** that the reference sensor (4) is mounted on a reference catheter (2) intended for insertion to a fixed position in the organ (12) or body structure to be visualized.

3. The system according to claim 1 or 2, **characterized in** that the probe (10) is equipped with at least three separate localization sensors (14).

4. The system according to any of claims 1-3, **characterized in** that the localization sensors (14) are arranged on the probe (10) in an essentially straight line that, when the probe is applied to the patient (6), is mainly perpendicular to the probe's contact surface with the patient.

5. The system according to claim 4, **characterized in** that tour separate reference sensors (14) are arranged on the reference catheter (2).

6. The system according to any of the preceding claims, **characterized in** that each reference and localization sensor (4, 14) has an ultrasound transceiver, and the localization unit (16) is arranged to determine the position of the probe (10), in relation to the reference sensor(s), from signals from the reference and localization sensors.

7. The system according to any of the previous claims, **characterized in** that the localization unit (16) is also arranged to determine the aiming direction and rotational position of the probe (10), in addition to its position, from signals from the reference and localization sensors (4, 14).

8. The system according to any of the previous claims, **characterized in** that image storage means (20) are arranged for storing the three-dimensional image of the organ (12).

9. The system according to claim 8, **characterized in** that an ECG device (22) is arranged to trigger the image generating unit (18) and the said image storage means (20) for storing images from ultrasonographs taken at a specific point in the heart cycle.
